# EUROPÄISCHE PATENTANMELDUNG

(11) **EP 4 450 013 A1**
(43) Veröffentlichungstag der Anmeldung: **23.10.2024**
(21) Anmeldenummer: 24170137.4
(22) Anmeldetag: 15.04.2024
(51) Int. Cl.: A61B 34/20, A61B 90/30

(54) **SYSTEM UND VERFAHREN ZUR LOKALISIERUNG UND IDENTIFIZIERUNG VON MEDIZINISCHEN ZIELOBJEKTEN**

(30) Priorität: 17.04.2023 DE 102023109620
(71) Anmelder: Karl Storz SE & Co. KG, 78532 Tuttlingen (DE)
(72) Erfinder: Brüderle, Klaus, 78532 Tuttlingen (DE)

(57) **Zusammenfassung**

System (10) zur Lokalisierung und Identifizierung von mehreren medizinischen Zielobjekten (12, 14), das System (10) aufweisend die mehreren medizinischen Zielobjekte (12, 14) und eine Messvorrichtung (16) zur Lokalisierung und Identifizierung der mehreren medizinischen Zielobjekte (12, 14), wobei die mehreren medizinischen Zielobjekte verschiedene Magnetfelder (20, 24) erzeugen, deren Position, Richtung und Quelle bestimmt werden kann. Es wird ferner ein Verfahren (50) zur Lokalisierung und Identifizierung von mehreren medizinischen Zielobjekten (12, 14) offenbart.

## Beschreibung

Die vorliegende Erfindung betrifft ein System zur Lokalisierung und Identifizierung von medizinischen Zielobjekten und ein Verfahren zur Lokalisierung und Identifizierung von medizinischen Zielobjekten.

Im Bereich der Medizintechnik werden üblicherweise Endoskope verwendet, um innenliegende Strukturen bildhaft zu erfassen. Damit können Diagnosen erstellt oder praktische Operationen durchgeführt werden. Dies beruht hauptsächlich darauf, dass die Sachverhalte optisch, also im sichtbaren Bereich, beurteilt und die Aktionen auf Basis dieser Beurteilung ausgewählt und ausgeführt werden.

Diese optischen Erfassungen betreffen die oberflächliche Situation. Diese müssen in Bezug zum Patienten beurteilt werden, deswegen ist eine Ortsbezug und eine Richtungserkennung wichtig. Während die wirksame Anwendung im Körper des Patienten stattfindet, erfolgt die Bedienung, insbesondere die Positionierung, von außen. Die Signale müssen daher die Körperbarriere überwinden, also von innen nach außen geführt werden. Dabei sind Übertragungsformen bevorzugt, die insbesondere Haut oder Knochen durchdringen können, also keinen extra Port benötigen.

Um die Richtung und die Position eines Zielobjekts räumlich zu erfassen, braucht es einen entsprechenden Signalgenerator, welcher mit dem Zielobjekt oder dem Teil des Zielobjekts in räumlich definierter Weise verbunden ist, insbesondere in einer festen Anordnung. Wenn sich das Zielobjekt im Körper befindet, wird das Signal vom Signalgenerator außerhalb des Körpers detektiert und entsprechend weiterverarbeitet und zum Beispiel für die Ortsbestimmung oder Navigation verwendet.

Ansätze zur Überwindung der Körperbarriere sind bereits aufgezeigt worden. So zeigt US 2014/0051985 A1 ein Zielfindungssystem, das ein chirurgisches Ziel identifiziert, wie z.B. einen Nierenstein, indem es einen Sender, wie z.B. eine magnetische Quelle, hinter oder neben dem chirurgischen Ziel anordnet und eine Schaltung einsetzt, um eine Achse zum Sender zu identifizieren und so eine Achse oder einen Weg zum chirurgischen Ziel zu definieren. Ein Array von Sensoren, die in einer äquidistanten, koplanaren Anordnung angeordnet sind, erfasst jeweils ein Signal, das den Abstand zum Emitter angibt. Ein Magnetwiderstandssensor, der einen variablen Widerstand erzeugt, reagiert auf den Abstand zu einer Magnetspule, die ein Magnetfeld aussendet. Ein gleiches Signal von jedem der koplanaren Sensoren zeigt die Positionierung auf einer Achse an, die durch einen Punkt in der Mitte der Sensoren und orthogonal zur Ebene verläuft.

Es ist eine Aufgabe der vorliegenden Erfindung ein verbessertes System und ein verbessertes Verfahren zur Lokalisierung und Identifizierung von medizinischen Zielobjekten aufzuzeigen,

Gemäß einem ersten Aspekt wird die Aufgabe gelöst durch ein medizinisches System zur Lokalisierung und Identifizierung von mehreren medizinischen Zielobjekten, das System aufweisend die mehreren medizinischen Zielobjekte und eine Messvorrichtung zur Lokalisierung und Identifizierung der mehreren Zielobjekte, wobei
- die mehreren medizinischen Zielobjekte ein erstes medizinisches Zielobjekt und ein zweites medizinisches Zielobjekt aufweisen,
- das erste medizinische Zielobjekt dafür ausgebildet ist, ein erstes Magnetfeld mit einem ersten zeitlichen Verlauf zu erzeugen,
- das zweite medizinische Zielobjekt dafür ausgebildet ist, ein zweites Magnetfeld mit einem zweiten zeitlichen Verlauf zu erzeugen, der vom ersten zeitlichen Verlauf verschieden ist, und
- die Messvorrichtung dafür ausgebildet ist, mittels eines Magnetfeldsensors Magnetfeldlinien des ersten Magnetfelds und des zweiten Magnetfelds hinsichtlich ihrer Richtung, ihrer Stärke und ihres zeitlichen Verlaufs zu detektieren, und mittels einer Verarbeitungsvorrichtung aus der detektierten Richtung und Stärke eine Position und Orientierung des jeweiligen Zielobjekts zu ermitteln, aus dem zeitlichen Auftreten die ermittelte Position und Orientierung einem der medizinischen Zielobjekte zuzuordnen und die Position und Orientierung für das zugeordnete medizinische Zielobjekt auszugeben.

Ein Vorteil dieser Lösung ist, dass mehr als nur ein Kommunikationskanal aufgebaut werden kann. Konkret, es können die Informationen, also Position und Orientierung, von mehreren medizinischen Zielobjekten erfasst und jeweils eindeutig einem der medizinischen Zielobjekte zugeordnet werden. Somit kann nun die Arbeit mit mehreren Zielobjekten verfolgt und überwacht werden.

Das genannte zeitliche Auftreten wird dabei über den Verlauf eines der Magnetfelder detektiert. Dafür ist es bevorzugt, dass mehrere Änderungen über den Magnetfeldsensor erfasst werden, so dass aus den detektierten Änderungen ein Rückschluss gezogen werden kann, welches der Zielobjekte diese Änderungen verursacht. Dafür hat jedes Zielobjekt insbesondere eine eigene Signatur, Taktung, Frequenz, Intervalle, Taktsequenz oder Amplitudenvarianz. Der Fachmann weiß, in Kenntnis der zu erwartenden Form des Nutzsignals, wie viele Samples benötigt werden, um ein Nutzsignal von einem anderen Nutzsignal zu unterscheiden, so dass auf diesen Aspekt hier nicht näher eingegangen wird.

Die vorgeschlagene Lösung bietet zudem weitere Vorteile. Da ist zum einen ein gutes Signal-Rauschverhältnis. Zwar sind die allgemeinen Rahmenbedingungen des Einsatzes so, dass das zu erwartende potenzielle Störsignal höher als das Nutzsignal ist. Die vorgeschlagene Lösung ermöglich aber trotz der ungünstigen Rahmenbedingungen eine gute Erkennung des Nutzsignals. Das gesuchte Nutzsignal kann zudem von anderen Umgebungssignalen isoliert werden, damit diese überlagerten Einflüsse geringgehalten werden. Zudem passieren magnetische Signale körperliche Barrieren, wie Haut und Knochen, ohne Informationsverlust und werden dadurch nicht oder nur vernachlässigbar beeinflusst oder gestört.

Die Erkennung erfolgt außerdem "stateless" und atomar. Es ist also keine Kenntnis oder Berücksichtigung eines vorherigen Verlaufs von Position und/oder Orientierung erforderlich. Dadurch unterscheiden sich die vorgeschlagene Lösung von anderen Verfahren, die die Position über die Addition der Bewegungswege des Zielobjekts erfassen. Dabei werden die Verlagerungen kontinuierlich über Beschleunigungssensoren erfasst und verarbeitet. Hier ist die Kenntnis der lückenlosen Sequenz der Bewegungsstrecken notwendig.

Das Magnetfeld des jeweiligen Zielobjekts ist in seiner räumlichen Ausprägung bekannt. In Kenntnis dieser Ausprägung kann aus der gemessenen Richtung und Stärke dann ein Rückschluss gezogen werden, an welcher Stelle des Magnetfelds sich der Magnetfeldsensor befindet. Daraus wiederum kann ermittelt werden, wo sich das Zielobjekt, insbesondere ein definierter Referenzpunkt der Zielobjekts,

Das Magnetfeld des jeweiligen Zielobjekts ist bei einigen Ausgestaltungen ein- und ausschaltbar, bei anderen bevorzugten Ausgestaltungen wird es durch eine Magnetspule erzeugt und kann durch eine Steuerung des Stromflusses durch die Magnetspule gesteuert werden. Bei einer solchen Ausgestaltung ist die Magnetspule über elektrische Impulse ansteuerbar.

Das Nutzsignal ist ein magnetisches Signal, genauer sind die Feldlinien, insbesondere die Feldlinienhüllkurve des Magnetfelds, das Nutzsignal. Dieses Nutzsignal hat somit eine elliptische Form, ist also ein Raumsignal, das hinsichtlich Stärke, Richtung und Position bestimmt ist. Der Magnetfeldsensor nutzt den magnetischen Teil, um das magnetische Raumsignal (X, Y, Z) zu detektieren. Dabei kann man sich das Raumsignal wie einen dreidimensionalen Körper vorstellen, der diese elliptische Form, also die Körperform des Signals, repräsentiert. Man kann sich ferner vorstellen, dass diese elliptische Form aus vielen kleinen Segmenten gebildet ist, die sich jeweils als Vektor beschreiben lassen. Indem man diese Vektoren bzw. Richtungsvektoren identifiziert, kann man die Position und Orientierung des jeweiligen Zielobjekts ermitteln.

Dadurch ist die Aufgabe vollständig gelöst.

Bei einer bevorzugten Ausgestaltung werden die Magnetfelder jeweils durch eine impulsartige Ansteuerung erzeugt.

Der Magnetfeldsensor reagiert auf alle magnetischen Einflüsse, wie Erdmagnetfeld, laufende Motoren, Stahlträger usw. Das sind aber statische oder zumindest definiert dynamische Signalformen (50Hz oder höher). Die Ansteuerung durch Impulse, insbesondere die Ansteuerung einer Magnetspule, kann hier so verwendet werden, dass diese Umgebungssignale mittels einer Auswertefilterung vom Nutzsignal separiert werden können. Konkret

Zudem kann diese Impulssteuerbarkeit, also insbesondere ein Ein- und Ausschalten auch so gestaltet werden, dass die resultierenden Nutzsignale überladen werden können. Es ist dann im Steuertakt eine Quellenkennung vorhanden. Diese Quellenkennung ist eine aufgeprägte Meta-Information. Dadurch wird die gewünschte Dualität von, einerseits, einer Raumsignalinformation für Richtung und Position und, andererseits, einer Metasignalinformation für Kanal- oder Quellkennung und zusätzlich zum zeitlichen Verlauf erreicht. Er ist demnach eine digitale Informationsübertragung von der Quelle, also dem Zielobjekt, zum Empfänger, also der Messvorrichtung. Der Steuertakt kann auch über der Zeit in seiner Frequenz und/oder Amplitude verändert werden, so dass eine bestimmte Quelle alternativ oder zusätzlich auch anhand einer Veränderung des Magnetfelds bzgl. Frequenz und/oder Amplitude über der Zeit identifiziert werden kann.

Bei einer bevorzugten Ausgestaltung werden die Magnetfelder jeweils durch eine wellenförmige Ansteuerung erzeugt, insbesondere durch eine sinusförmige Ansteuerung.

Hier besteht einerseits die Möglichkeit, eine Quelle anhand der von ihr verwendeten Wellenform zu identifizieren, z.B. Sinusform, Rechteckform, Dreieckform, Sägezahnform, etc. Dazu wird der zeitliche Verlauf des Raumsignals an einer Stelle im Raum gemessen, um aus dem gemessenen zeitlichen Verlauf, auf die Quelle zurückschließen zu können. Alternativ oder zusätzlich kann auch die Frequenz und/oder die Amplitude der wellenförmigen Ansteuerung gewählt werden und später aus dem zeitlichen Verlauf des Raumsignals bestimmt werden, um auf die Quelle zurückschließen zu können. Alternativ oder zusätzlich kann auch die Frequenz und/oder die Amplitude der wellenförmigen Ansteuerung im zeitlichen Verlauf verändert werden. Es wird dann aus dem zeitlichen Verlauf des Raumsignals die Veränderung von Frequenz und/oder Amplitude ermittelt, um auf die Quelle zurückschließen zu können. So kann auf viele verschiedene Weisen zwischen verschiedenen Signalquellen differenziert werden, wobei die Merkmale Form, Frequenz, Amplitude, Formänderung, Frequenzänderung und Amplitudenänderung in beliebiger Kombination oder in Alleinstellung zur Identifikation einer Quelle verwendet werden können.

Bei einer weiteren bevorzugten Ausgestaltung erzeugt das erste Zielobjekt das erste Magnetfeld periodisch mit einer ersten Frequenz, erzeugt das zweite Zielobjekt das zweite Magnetfeld periodisch mit einer zweiten Frequenz und die weist Messvorrichtung ein variables Bandpassfilter auf, dessen Mittenfrequenz wahlweise auf die erste Frequenz und die zweite Frequenz eingestellt werden kann.

Grundsätzlich kann durch eine zeitliche Trennung, wann das erste Zielobjekt und das zweite Zielobjekt das jeweilige Magnetfeld erzeugen, sichergestellt werden, dass die Magnetfelder gut unterschieden werden können. Bei dieser Ausgestaltung ist es aber sogar möglich, die Magnetfelder zu unterscheiden, wenn sie sich zeitlich überlagern. Der Bandpassfilter erzielt, dass jeweils ein Signal eines gewünschten Magnetfelds dominant erfasst wird.

Bei einer weiteren bevorzugten Ausgestaltung wird jedes Magnetfeld mit einem jeweiligen Strom erzeugt wird, der ein Rechtecksignal oder ein Sägezahnsignal oder ein Dreiecksignal oder besonders bevorzugt ein Sinussignal ist.

Diese Ausgestaltung ermöglicht eine gute Trennung zwischen den Magnetfeldern.

Bei einer weiteren bevorzugten Ausgestaltung ist der Magnetfeldsensor als Bauteil eines Tablets ausgeführt, insbesondere als Teil eines Gyroskopsensors des Tablets.

Auf diese Weise kann eine kostengünstige Lösung erzielt werden, die sich zudem nahtlos in die bereits vorhandene Nutzung eines Tablets, z.B. für die Sichtung und Eingabe von Daten, einfügt.

Bei einer weiteren bevorzugten Ausgestaltung ist das erste Zielobjekt an einer im Raum ortsfesten Stelle angeordnet ist, insbesondere an einem Operationstisch.

Damit ist ein Ortsbezug des Zielobjekts zur Arbeitsumgebung herstellbar. Dazu kann das Zielobjekt außerhalb des Körpers, insbesondere in einem Operationstisch, bevorzugt in einem Fuß des Operationstischs, eingebaut sein. Das Material ist dann insbesondere V2A- oder Edelstahl. Ein derart fix positioniertes Zielobjekt wird bevorzugt zum Ortsabgleich verwendet, wie er beispielsweise vom Differential-GPS bekannt ist.

Bei einer weiteren bevorzugten Ausgestaltung beträgt der Abstand zwischen der Messvorrichtung und jedem der Zielobjekte höchstens 3m, beträgt bevorzugt zwischen 0,5m und 2,5m und beträgt besonders bevorzugt zwischen 1m und 2m.

Aufgrund des geringen Abstands, also durch die kurze Wirkdistanz, ist die benötigte Signalintensität gering. Diese ist um Größenordnungen geringer als beispielsweise in einer MRT-Vorrichtung bzw. einem Magnetresonanztomographen.

Bei einer weiteren bevorzugten Ausgestaltung weist die Messvorrichtung mehrere Magnetfeldsensoren auf, die voneinander beabstandet sind und demselben Funktionsprinzip unterliegen, und ist die Messvorrichtung dafür ausgebildet, Position und Orientierung des jeweiligen Zielobjekts, wie sie von den einzelnen Magnetfeldsensoren ermittelt werden, gegeneinander auf Plausibilität zu prüfen und/oder durch eine Mittelung die Genauigkeit von Position und Orientierung zu verbessern.

Bei dieser Ausgestaltung wird das Signal an mehreren unterschiedlichen Positionen empfangen und ausgewertet. Das heißt, dass zwei oder mehr Sensoren werten die Raumellipse des Magnetfeldes eines medizinisches Zielobjekts aus und das Ergebnis sollte zumindest näherungsweise dieselben Positionsdaten des zugehörigen medizinischen Zielobjekts ergeben. Dies bedeutet, dass man hat eine Verifizierung bzw. Plausibilitätsprüfung der Erfassung zur Laufzeit hat und somit eine höhere Zuverlässigkeit. Zusätzlich oder alternativ kann durch eine Mittelung der Messwert die Genauigkeit verbessert werden. Dadurch, dass mehrere Messungen an verschiedenen Positionen durchgeführt werden, kann man auch von einer Zwei-Faktor-Auswertung sprechen, die die Genauigkeit erhöht.

Bei einer weiteren bevorzugten Ausgestaltung ist das zweite Zielobjekt ortsfest an einem Endoskop angeordnet.

Dadurch kann die eingangs angesprochener häufiger Verwendung eines Endoskops in vorteilhafter Weise verbessert werden.

Gemäß einem zweiten Aspekt wird die Aufgabe gelöst durch ein Verfahren zur Lokalisierung und Identifizierung von mehreren medizinischen Zielobjekten, das Verfahren mit den Schritten:
- Erzeugen eines ersten Magnetfelds mit einem ersten zeitlichen Verlauf durch ein erstes medizinisch Zielobjekt;
- Erzeugen eines zweiten Magnetfelds mit einem zweiten zeitlichen Verlauf, der vom ersten zeitlichen Verlauf verschieden ist, durch ein zweites medizinisch Zielobjekt;
- Detektieren, mittels eines Magnetfeldsensors, von Magnetfeldlinien des ersten Magnetfelds und des zweiten Magnetfelds hinsichtlich ihrer Richtung, ihrer Stärke und ihres zeitlichen Verlaufs,
- Ermitteln, mittels einer Verarbeitungsvorrichtung, einer Position und Orientierung des jeweiligen medizinischen Zielobjekts aus der Richtung und der Stärke,
- Zuordnen, mittels der Verarbeitungsvorrichtung, der ermittelten Position und Orientierung zu einem der medizinisch Zielobjekte anhand des zeitlichen Verlaufs; und
- Ausgeben der Position und Orientierung für das zugeordnete medizinische Zielobjekt.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in den Zeichnungen dargestellt und wird in der nachfolgenden Beschreibung näher erläutert. Es zeigen:
- Fig. 1: eine Ausführungsform eines medizinischen Systems zur Lokalisierung und Identifizierung von mehreren Zielobjekten;
- Fig. 2: eine Ausführungsform eines Verfahrens zur Lokalisierung und Identifizierung von mehreren medizinischen Zielobjekten; und
- Fig. 3: eine Ausführungsform einer Messvorrichtung mit mehrere Magnetfeldsensoren.

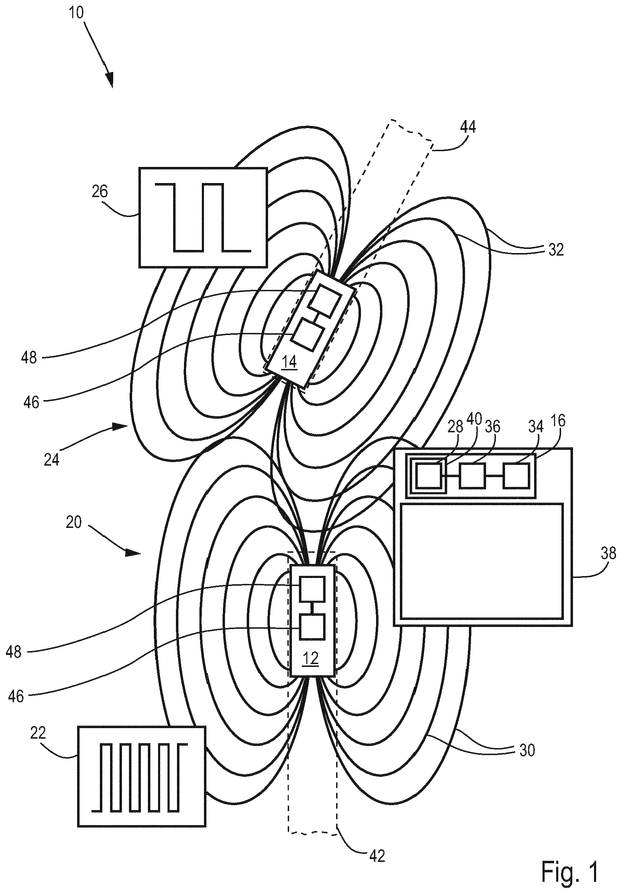

Fig. 1 zeigt eine Ausführungsform eines medizinisches Systems 10 zur Lokalisierung und Identifizierung von mehreren medizinischen Zielobjekten 12, 14. Zu dem System 10 gehören die mehreren Zielobjekte 12, 14 und eine Messvorrichtung 16 zur Lokalisierung und Identifizierung der mehreren Zielobjekte 12, 14.

Konkret weisen die mehreren Zielobjekte 12, 14 ein erstes Zielobjekt 12 und ein zweites Zielobjekt 14 auf. Dabei ist das erste Zielobjekt 12 dafür ausgebildet, ein erstes Magnetfeld 20 mit einem ersten zeitlichen Verlauf 22 zu erzeugen, und ist das zweite Zielobjekt 14 dafür ausgebildet, ein zweites Magnetfeld 24 mit einem zweiten zeitlichen Verlauf 26 zu erzeugen, der vom ersten zeitlichen Verlauf 22 verschieden ist

Die Messvorrichtung 16 ist dafür ausgebildet, mittels eines Magnetfeldsensors 28 der Messvorrichtung 16 Magnetfeldlinien 30, 32 des ersten Magnetfelds 20 und des zweiten Magnetfelds 24 hinsichtlich ihrer Richtung, ihrer Stärke und ihres zeitlichen Verlaufs zu detektieren.

Mittels einer Verarbeitungsvorrichtung 34 der Messvorrichtung 16 wird dann aus der detektierten Richtung und Stärke eine Position und Orientierung des jeweiligen Zielobjekts 12, 14 zu ermittelt. Dies ist möglich, da die Form des Magnetfelds 20, 24, das von einem Zielobjekt 12, 14 erwartet wird, bekannt ist. Kennt man die Form des Magnetfelds 20, 24, kann daraus geschlossen werden, an welcher Stelle dieser Form sich der Magnetfeldsensor 28 befindet, so dass darauf wiederum auf den Ursprung des Magnetfelds 20, 24, also auf das Zielobjekt 12, 14 bzw. einen Referenzpunkt des Zielobjekts 12, 14, relativ zum Magnetfeldsensor 28 geschlossen werden kann.

Aus dem zeitlichen Auftreten des Magnetfelds 20, 24 wird von der Verarbeitungsvorrichtung 34 die ermittelte Position und Orientierung einem der Zielobjekte 12, 14 zugeordnet. Dies ist möglich, da die Zielobjekte 12, 14 ihr jeweiliges Magnetfeld mit unterschiedlichen zeitlichen Verläufen 22, 26 erzeugen. Wird beispielsweise das erste Magnetfeld 20 schnell ein- und ausgeschaltet und wird das zweite Magnetfeld 24 langsam ein- und ausgeschaltet, lassen sich die Magnetfelder aufgrund des zeitlichen Verlaufs 22, 26 der resultierenden Magnetfelder 20, 24 unterscheiden. Dafür können die Magnetfelder 20, 24 jeweils durch eine Magnetfeldspule 46 erzeugt werden, die von einer Ansteuereinheit 48 impulsartig angesteuert wird.

Schließlich ist die Verarbeitungsvorrichtung 34 dafür ausgebildet, die Position und Orientierung für das zugeordnete Zielobjekt 12, 14 auszugeben. Mit dieser Information kann insbesondere das jeweilige Zielobjekt 12, 14 virtuell dargestellt werden, zum Beispiel auf einem Bildschirm oder einer VR-Brille. Dadurch kann der Benutzer die räumliche Situation intuitiv erfassen, obwohl der Benutzer keine Sichtlinie auf das jeweilige Zielobjekt 12, 14 hat.

Bei der hier gezeigten Ausführungsform erzeugt das erste medizinische Zielobjekt 12 das erste Magnetfeld 20 periodisch mit einer ersten Frequenz und erzeugt das zweite medizinische Zielobjekt 14 das zweite Magnetfeld 24 periodisch mit einer zweiten Frequenz. Die Messvorrichtung 16 weist ein variables Bandpassfilter 36 auf, dessen Mittenfrequenz wahlweise auf die erste Frequenz und die zweite Frequenz eingestellt werden kann. So ist es möglich, die Erfassung der beiden Magnetfelder 20, 24 zu trennen, selbst wenn sie sich zeitweise überlagern.

Der Magnetfeldsensor 28 ist hier als Bauteil eines Tablets 38 ausgeführt, insbesondere als Teil eines Gyroskopsensors 40 des Tablets 38. Zudem ist das erste Zielobjekt 12 hier an einer im Raum ortsfesten Stelle angeordnet, insbesondere an einem Operationstisch 42, der hier gestrichelt angedeutet ist. Das zweite Zielobjekt 14 ist ortsfest an einem Endoskop 44 angeordnet, das ebenfalls gestrichelt angedeutet ist.

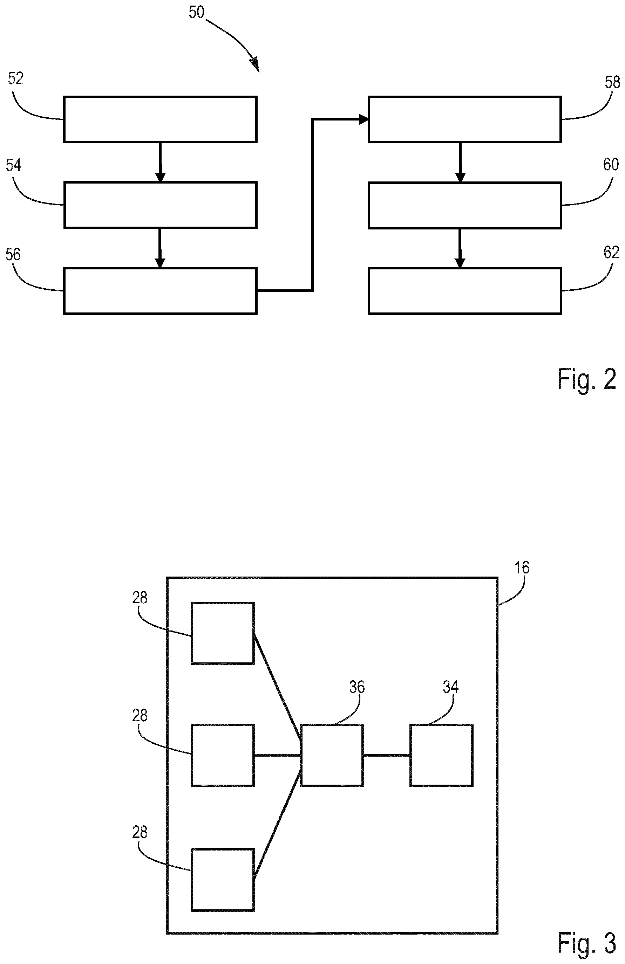

Fig. 2 zeigt eine Ausführungsform eines medizinischen Verfahrens 50 zur Lokalisierung und Identifizierung von medizinischen Zielobjekten 12, 14. Das Verfahren 50 beginnt mit dem Erzeugen 52 eines ersten Magnetfelds 20 mit einem ersten zeitlichen Verlauf 22 durch ein erstes medizinischen Zielobjekt 12. Zudem erfolgt ein Erzeugen 54 eines zweiten Magnetfelds 24 mit einem zweiten zeitlichen Verlauf 26, der vom ersten zeitlichen Verlauf 22 verschieden ist, durch ein zweites medizinischen Zielobjekt 14.

Es folgt der Schritt des Detektierens 56, mittels eines Magnetfeldsensors 28, von Magnetfeldlinien 30, 32 des ersten Magnetfelds 2ß und des zweiten Magnetfelds 24 hinsichtlich ihrer Richtung, ihrer Stärke und ihres zeitlichen Verlaufs 22, 26. In einem weiteren Schritt erfolgt, mittels einer Verarbeitungsvorrichtung 34, ein Ermitteln 58 einer Position und Orientierung des jeweiligen Zielobjekts 12, 14 aus der Richtung und der Stärke.

Im nächsten Schritt erfolgt ein Zuordnen 60, mittels der Verarbeitungsvorrichtung 34, der ermittelten Position und Orientierung zu einem der Zielobjekte 12, 14 anhand des zeitlichen Verlaufs. Schließlich erfolgt das Ausgeben 62 der Position und Orientierung für das zugeordnete Zielobjekt 12, 14.

Fig. 3 zeigt eine Ausführungsform einer Messvorrichtung 16 mit mehreren Magnetfeldsensoren 28, die voneinander beabstandet sind und demselben Funktionsprinzip unterliegen. Bei dieser Ausführungsform ist die Messvorrichtung 16 dafür ausgebildet, Position und Orientierung des jeweiligen Zielobjekts 12, 14, wie sie von den einzelnen Magnetfeldsensoren 42 ermittelt werden, gegeneinander auf Plausibilität zu prüfen und/oder durch eine Mittelung die Genauigkeit von Position und Orientierung zu verbessern.

## Patentansprüche

1. Medizinisches System (10) zur Lokalisierung und Identifizierung von mehreren medizinischen Zielobjekten (12, 14), das System (10) aufweisend die mehreren medizinischen Zielobjekte (12, 14) und eine Messvorrichtung (16) zur Lokalisierung und Identifizierung der mehreren medizinischen Zielobjekte (12, 14), wobei
- die mehreren medizinischen Zielobjekte (12, 14) ein erstes medizinisches Zielobjekt (12) und ein zweites medizinisches Zielobjekt (14) aufweisen,
- das erste medizinische Zielobjekt (12) dafür ausgebildet ist, ein erstes Magnetfeld (20) mit einem ersten zeitlichen Verlauf (22) zu erzeugen,
- das zweite medizinische Zielobjekt (14) dafür ausgebildet ist, ein zweites Magnetfeld (24) mit einem zweiten zeitlichen Verlauf (26) zu erzeugen, der vom ersten zeitlichen Verlauf (22) verschieden ist, und
- die Messvorrichtung (16) dafür ausgebildet ist, mittels eines Magnetfeldsensors (28) Magnetfeldlinien (30, 32) des ersten Magnetfelds (20) und des zweiten Magnetfelds (24) hinsichtlich ihrer Richtung, ihrer Stärke und ihres zeitlichen Verlaufs zu detektieren, und mittels einer Verarbeitungsvorrichtung (34) aus der detektierten Richtung und Stärke eine Position und Orientierung des jeweiligen Zielobjekts (12; 14) zu ermitteln, aus dem zeitlichen Auftreten die ermittelte Position und Orientierung einem der Zielobjekte (12; 14) zuzuordnen und die Position und Orientierung für das zugeordnete medizinischen Zielobjekt (12; 14) auszugeben.

2. Medizinisches System nach Anspruch 1, wobei die Magnetfelder (20, 24) jeweils durch eine impulsartige Ansteuerung erzeugt werden.

3. Medizinisches System nach Anspruch 1, wobei die Magnetfelder jeweils durch eine wellenförmige Ansteuerung erzeugt werden, insbesondere durch eine sinusförmige Ansteuerung.

4. Medizinisches System nach einem der vorhergehenden Ansprüche, wobei das erste Medizinische Zielobjekt (12) das erste Magnetfeld (20) periodisch mit einer ersten Frequenz erzeugt, das zweite Medizinische Zielobjekt (14) das zweite Magnetfeld (24) periodisch mit einer zweiten Frequenz erzeugt und die Messvorrichtung (16) ein variables Bandpassfilter (36) aufweist, dessen Mittenfrequenz wahlweise auf die erste Frequenz und die zweite Frequenz eingestellt werden kann.

5. Medizinisches System nach Anspruch 4, wobei jedes Magnetfeld (20, 24) mit einem jeweiligen Strom erzeugt wird, der ein Rechtecksignal oder ein Sägezahnsignal oder ein Dreiecksignal oder besonders bevorzugt ein Sinussignal ist.

6. Medizinisches System nach einem der vorhergehenden Ansprüche, wobei der Magnetfeldsensor (28) als Bauteil eines Tablets (38) ausgeführt ist, insbesondere als Teil eines Gyroskopsensors (40) des Tablets (38).

7. Medizinisches System nach einem der vorhergehenden Ansprüche, wobei das erste Medizinische Zielobjekt (12) an einer im Raum ortsfesten Stelle angeordnet ist, insbesondere an einem Operationstisch (42).

8. Medizinisches System nach einem der vorhergehenden Ansprüche, wobei der Abstand zwischen der Messvorrichtung (16) und jedem der Medizinische Zielobjekte (12, 14) höchstens 3m beträgt, bevorzugt zwischen 0,5m und 2,5m beträgt und besonders bevorzugt zwischen 1m und 2m beträgt.

9. Medizinisches System nach einem der vorhergehenden Ansprüche, wobei die Messvorrichtung (16) mehrere Magnetfeldsensoren (28) aufweist, die voneinander beabstandet sind und demselben Funktionsprinzip unterliegen, und die Messvorrichtung (16) dafür ausgebildet ist, Position und Orientierung des jeweiligen Medizinischen Zielobjekts (12, 14), wie sie von den einzelnen Magnetfeldsensoren (42) ermittelt werden, gegeneinander auf Plausibilität zu prüfen und/oder durch eine Mittelung die Genauigkeit von Position und Orientierung zu verbessern.

10. Medizinisches System nach einem der vorhergehenden Ansprüche, wobei das zweite Zielobjekt (14) ortsfest an einem Endoskop (44) angeordnet ist.

11. Verfahren (50) zur Lokalisierung und Identifizierung von mehreren Medizinischen Zielobjekten (12, 14), das Verfahren mit den Schritten:
- Erzeugen (52) eines ersten Magnetfelds (20) mit einem ersten zeitlichen Verlauf (22) durch ein erstes Medizinisches Zielobjekt (12);
- Erzeugen (54) eines zweiten Magnetfelds (24) mit einem zweiten zeitlichen Verlauf (26), der vom ersten zeitlichen Verlauf (22) verschieden ist, durch ein zweites Medizinisches Zielobjekt (14);
- Detektieren (56), mittels eines Magnetfeldsensors (28), von Magnetfeldlinien (30, 32) des ersten Magnetfelds (20) und des zweiten Magnetfelds (24) hinsichtlich ihrer Richtung, ihrer Stärke und ihres zeitlichen Verlaufs (22, 26),
- Ermitteln (58), mittels einer Verarbeitungsvorrichtung (34), einer Position und Orientierung des jeweiligen Medizinischen Zielobjekts (12; 14) aus der Richtung und der Stärke,
- Zuordnen (60), mittels der Verarbeitungsvorrichtung (34), der ermittelten Position und Orientierung zu einem der Medizinischen Zielobjekte (12, 14) anhand des zeitlichen Verlaufs (22, 26); und
- Ausgeben (62) der Position und Orientierung für das zugeordnete Medizinische Zielobjekt (12; 14).
